(19) Europäisches Patentamt / European Patent Office / Office européen des brevets

(11) **EP 3 031 432 B1**

(12) **EUROPEAN PATENT SPECIFICATION**

(45) Date of publication and mention
of the grant of the patent:
**06.06.2018  Bulletin 2018/23**

(51) Int Cl.:
*A61F 13/84* (2006.01)    *A61B 6/00* (2006.01)
*A61F 13/20* (2006.01)

(21) Application number: **15198474.7**

(22) Date of filing: **08.12.2015**

(54) **TAMPON TEST METHOD AND APPARATUS**

TAMPONTESTVERFAHREN UND -VORRICHTUNG

PROCÉDÉ ET APPAREIL DE TEST DE TAMPON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:  **09.12.2014  US 201414564780**

(43) Date of publication of application:
**15.06.2016  Bulletin 2016/24**

(73) Proprietor: **Johnson and Johnson GmbH
41470 Neuss (DE)**

(72) Inventors:
• **ACTON, Paul
Halboro, PA Pennsylvania 19040 (US)**
• **HOU, Mari
Basking Ridge, NJ New Jersey 07920 (US)**

(74) Representative: **Carpmaels & Ransford LLP
One Southampton Row
London WC1B 5HA (GB)**

(56) References cited:
WO-A2-2008/021173      US-A1- 2003 147 490
US-A1- 2005 273 036      US-A1- 2011 060 548
US-A1- 2012 321 040

**Description**

**Field of the Invention**

The present invention relates to a method of analyzing internal sanitary protection articles in order to determine the effect of fiber density on the fluid absorbent properties of the article. It is especially helpful in understanding how absorbency of a compressed article such as a tampon can be affected by fiber density and fiber density gradients formed by compression and/or manufacturing process.

**Description of the Prior Art**

Tampons are absorbent articles and formed in order to absorb menstrual fluid before it exits the vagina. While tampons are efficient in offering protection to the wearer, tampons can fail, which results in leakage unto the users undergarment. There are three basic reasons for failure: the tampon is saturated with fluid and cannot hold any more, the user has a gush of fluid due to fluid pooling in the body and a sudden intra-abdominal pressure occurs (either due to changes in body position or stress event such as coughing, laughing, or sneezing) which the tampon cannot quickly absorb or the fluid has managed to by-pass the tampon and exit the body without contacting the absorbent material. The first mode of leakage can be mitigated by changing the tampon; the second mode of leakage can be improved by the inherent properties of the tampon and the third is the subject of structure and material improvements.

The second type of leakage is referred to as "early leakage," where women see the tampon as "half-full" and still leaks. Early leakage can be avoided by developing tampons that quickly absorb the fluid and keeps the fluid in after uptake. o.b.® tampons were developed to have a fiber density gradient with lower fiber density on the outside to quickly move fluid in and a higher fiber density in the inside to secure the fluid within its core. Understanding and quantifying this fiber density and fiber density gradient of tampons and how it affects its ability to manage menstrual fluid is important for developing tampons with superior leakage protection.

Standard ways to calculate the fiber density of a tampon, where overall fiber density is derived from the mass and volume, are limited as they only provide the fiber density of the tampon as a whole rather than the fiber density gradient through the cross-section of the tampon. Other methodologies include single slice 2-D X-ray scanning and impregnating the tampon in epoxy and slicing the tampon to visualize its cross-section. These methods are also limited as it only provides a qualitative understanding rather than a quantitative measurement of the fiber density of the tampon at its outer and inner sections.

US 6839402 purports to disclose a radiological system for analyzing an absorbent article. The system can include computed tomography (CT) and measures the liquid in an absorbent article. Examples of diapers and sanitary napkins were analyzed. A visualization of the liquid within an article can be displayed as can just the liquid alone can be displayed.

US 7166085 purports to disclose an in vitro testing device to study the placement of a tampon into a receptacle representing relevant in vivo anatomical and physical characteristics. It discloses a coronal MRI image of a user having a tampon in the vagina, inserted by the user using a typical commercial tampon and applicator system.

US 20120277710 purports to disclose methods for making absorbent members having fiber density profile. A micro CT Scan procedure is described under Test Methods, which is used to quantitatively measure the fiber density profile throughout the thickness of the absorbent article non-invasively. The section of the absorbent article tested is preferably free from embossments and apertures and cut from the article in a way that doesn't disrupt the thickness or fiber density of the section. The micro-computed tomography system used in the test methods is μCT 40, ID#4286, Scanco Medical AG.

This invention relates to the use of X-ray CT (computed tomography) scanning technology and software to understand the fiber density and the fiber density gradient of tampon products in quantitative measures. This invention allows for improved analysis of a tampon, especially as it is exposed to fluid in a simulated anatomical environment.

SUMMARY OF THE INVENTION

The invention is defined in the appended claims. In one aspect of the invention, an apparatus for in vitro testing of an absorbent article (tampon) includes a radiolucent pressure chamber, a radiological device, and a computing device. The radiolucent pressure chamber includes a radiolucent simulated body cavity arranged and configured to accommodate the absorbent article, a test liquid port, and a pressure control system. The radiolucent simulated body cavity comprises a vaginal model. The radiological device is disposed to collect radiological data from the absorbent article, and it includes a radiolucent test device support capable of accommodating the radiolucent pressure chamber, and at least one radio scanning element rotatable about the radiolucent test device support. The computing device is operatively connected to the radiological device and has a program for analyzing the data collected by the radiological device.

In another aspect of the invention, a method for in vitro testing of an absorbent article (tampon) includes the steps of inserting the absorbent article into a radiolucent simulated body cavity, placing the radiolucent simulated body cavity into a radiolucent pressure chamber, placing the radiolucent pressure chamber into a radiological device, pressurizing the radiolucent pressure chamber to a predetermined pressure, providing test liquid through the test liquid port and to the absorbent article, acquiring data relating to the absorbent article and the test liquid via the radiological device and an associated computing device, and analyzing the acquired data. The radiolucent simulated body cavity comprises a vaginal model. The radiological pressure chamber includes a simulated body cavity arranged and configured to accommodate the absorbent article, a test liquid port, and a pressure control system. The radiolucent test device has at least one radio scanning element rotatable about radiolucent test device support having the pressure chamber disposed thereon.

BRIEF DESCRIPTION OF THE DRAWINGS

Fig. 1 is a perspective view of a CT scanning system according to one embodiment of the present invention.
Figs. 2A-C include three views of a test cylinder useful in the present invention. Fig. 2A is a plan view of the test cylinder; Fig. 2B is an end view of the test cylinder; and Fig. 2C is a cross-section of the test cylinder taken along line C-C of Fig. 2A.
Fig. 3 is an exemplary CT scan image of a tampon with Regions of Interest identified by circles.
Fig. 4 is a graph of the correlation of fibrous pellet density to x-ray density in Hounsfield Units.
Fig. 5 is a graph comparing the fiber density of various ROIs of a commercial tampon against a similar tampon prototype.
Fig. 6 is a graph comparing the fiber density gradient of a commercial tampon against a similar tampon prototype.
Fig. 7 is a graph showing the x-ray intensity data, in a 2-dimensional view through the tampon, during test fluid absorption by the tampon.

DETAILED DESCRIPTION OF THE PREFERRED EMBODIMENTS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.

Understanding how fluid dynamically moves through a tampon is an important part of assessing tampon structure and function to improve the products. The main challenge with this understanding is that the fluid movement and distribution through a tampon cannot be visualized without advanced medical imaging technologies. We have devised a novel method to qualitatively understand how fluid moves through a tampon using an in vitro apparatus that simulates the vaginal environment.

This method improves on state-of-the-art scanning technology such as x-ray computed tomography ("CT") scanning in which absorbent articles may be scanned. In one improvement, tampons can be scanned inside a plastic vial extending over the end of the scanner bed. After scanning each tampon, the tampon fiber density can be measured from the CT scan images and corrected to Hounsfield Units ("HU") as described below.

In addition, the method takes advantage of the concepts in Hou et al., US Pat. App. Pub. No. US 2011-0200976 A1, describing a pressurized simulated testing device and method.

Thus, the inventive method employs an x-ray transparent, pressurizable container for a tampon. The container includes one or more environmental control ports and at least one liquid delivery port.

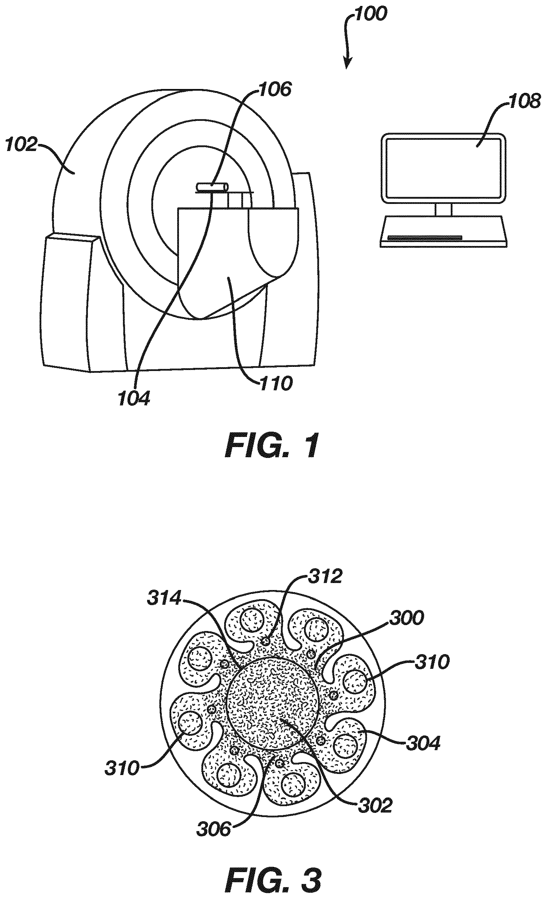

In one embodiment, shown in Fig. 1, a radiological system 100 includes a CT scanner 102 having at least one radio scanning element rotatable about a radiolucent test device support (test bed 104), a test container 106, and a computing system 108. The test container 106 is placed on the radiolucent test bed 104. The radiolucent test bed 104 is mounted on a track on a test bed support 110 to be movable into the CT scanner 102. The CT scanner 102 and test container 106 are interfaced with the computing system 108 to control the radiological system and to record data generated by the radiological system.

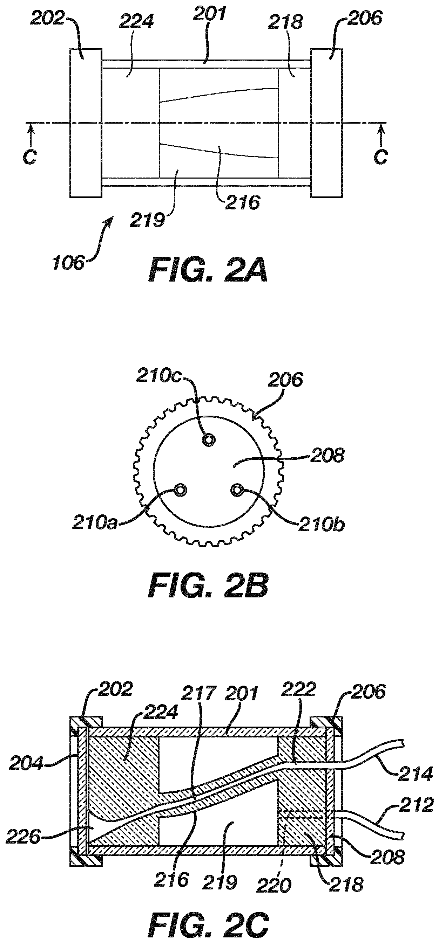

In the embodiment shown in Fig. 2, the test container 106 is a substantially cylindrical radiolucent pressure chamber. The test container 106 has a cylindrical tube 201, a first end cap comprising a first threaded ring 202 and a first cover plate 204 secured thereto, and it is removable for insertion of a test tampon (not shown), and a second end cap comprising a second threaded ring 206 and a second cover plate 208 having a plurality of ports 210. A first port 210a provides a pressurizing fluid, such as air, via a first conduit 212 a second port 210b for a pressure sensor (not shown), and a third port 210c for introduction of a test liquid via a second conduit 214 into the test container 106. Disposed within the test container 106 is a radiolucent simulated body cavity 216 (a simulated vagina having a lumen 217 in the present embodiment) that is arranged and configured to accommodate the test article (a tampon in the present embodiment). In the embodiment of Fig. 2, the radiolucent simulated body cavity 216 is connected to a first support 218 disposed adjacent the second cover plate 208 to maintain the simulated body cavity 216 in a predetermined position. The first support includes pressurization channels 220 connecting the first and second ports 210a, 210b to the interior 219 of the cylindrical tube 201 surrounding the simulated body cavity 216 and a test liquid channel 222 connecting the third port 210c to the lumen 217 of the simulated body cavity 216. A second support 224 is connected to the simulated body cavity 216 adjacent the first cover plate 204 and has an opening 226 connected to the lumen 217 for introduction of test devices into the simulated body cavity 216. The pressurizing fluid and test liquid are also controlled by the computing system 108.

Analytical methods:

CT Scanning for fiber density:

Current commercial o.b.® ProComfort tampons in Normal absorbency, 9-12 g made out of 75% GALAXY® rayon fibers (available from Kelheim Fibres GmbH, Kelheim, Germany) and 25% viscose rayon fibers (N=10) and a prototype tampon of "higher fiber density" made out of 100% GALAXY® rayon fibers in "Normal" absorbency, 9-12g (N=10) were placed on the radiolucent test bed 104 of an Inveon CT scanner (Siemens Medical Systems, Knoxville, TN), such as shown in Fig. 1 and imaged. Both tampon types have the same absorbency band of 9-12 grams with similar fiber compositions. They both have the same dimensional length of 48mm, but they differ in diameter. The current commercial o.b.® ProComfort tampon has a diameter of 13 mm, while the higher fiber density tampon has a diameter of 12 mm. To minimize the environmental effects on the tampon, the clear tampon polypropylene overwrap and the tampon removal string were kept in place during the scanning procedure.

Tampons were scanned inside a plastic 10 ml vial extending over the end of the scanner bed. CT scan images were taken from the tip of the tampon to the base, using the following settings: X-ray source 80 kVp, 500 μA, X-ray detector magnification low, binning 2, CCD size 3072 x 2048, exposure 800 ms/step, 360 rotation steps over 360 degrees. Images were reconstructed using a cone-beam filtered back projection algorithm on a Cobra multiprocessor system, into images of size $1536 \times 1536 \times 1024$, with isotropic voxels of size 53 μm.

After scanning each tampon, the tampon fiber density was measured from the CT scan images using PMOD software (PMOD Technologies Ltd, Zurich, Switzerland), and corrected to Hounsfield Units (HU) using scale factors derived from a CT scan of a small vial of water (air HU = -1000, water HU = 0). Fig. 3 is an example of such a CT scan image of the cross-section of a tampon 300 having a tampon core 302 and eight lobes 304 connected thereto via eight stems 306. Regions of interest (ROIs) were drawn a indicated by circles at each of the eight lobes (circles 310), eight stems (circles 312), and core (circle 314) of the tampon at the center transverse slice of the CT image. The lobe 304 of the tampon is defined as the outer part of the tampon that immediately contacts the body and fluid. The stem 306 is defined as the area in-between the lobe and the center of the tampon. The core 302 of the tampon is the center of the tampon. With the light background of Fig. 3, higher fiber density regions are relatively dark in the image, and lower fiber density regions are relatively light. The ROIs were taken in a standardized manner for each tampon, as identified in Figure 3.

The tampon fiber density was converted from HU units to the standard density units of grams per cubic centimeter (g/cc) using CT scans of pellets of known fiber density. Fiber pellets of two different materials were compressed at various pressures (1500-2500 psi) to generate a range of fiber density values. The pellets were weighed, and scanned in the CT machine, and the size of each pellet was measured from the images. A total of 30 pellets were imaged: fifteen were made with a uniform blend of 75% Galaxy rayon fibers and 25% Viscose rayon fibers (same as the ProComfort tampon) and fifteen were made with 100% Galaxy rayon fibers (same as the High Fiber density Prototype).

The pellet fiber density data in HU and g/cc units were plotted and a linear regression line was fit to the data. The regression equation was used to convert the HU fiber density data to g/cc data for the tampon analysis.

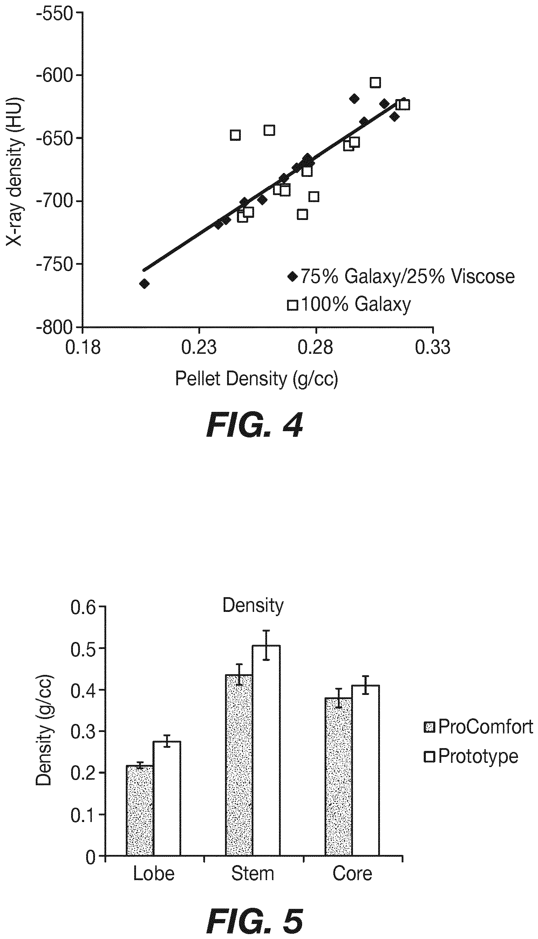

Figure 4 shows the pellet densities measured from the known mass and size of each pellet, and the X-ray density derived from ROIs placed on the CT images. The 75% Galaxy/25% Viscose pellets appeared to have higher variability than the 100% Galaxy devices, although both types showed good correlations with X-ray density. The tampon densities can be derived from the CT HU measurement, using the following conversion:

$$Density \ (g \ / \ cc) = (HU + 1008)/1224$$

Table 1, below, summarizes the average fiber density of the eight lobes, eight stems, and core for the o.b.® ProComfort tampon and the "high fiber density" tampon Prototype. Figure 5 shows the fiber density profile of the o.b.® ProComfort tampon versus the Prototype tampon at the lobe, stem, and core. The fiber density profile for both tampons shows lower fiber density at the lobe and higher fiber density at the stem and core, which allows the fluid to move quickly away from the outer surface of the tampon to the inner core.

**Table 1: Fiber density at 3 different locations in the central slice of each tampon**

| Tampon type | Lobe Fiber density (g/cc) | Stem Fiber density (g/cc) | Core Fiber density (g/cc) |
|---|---|---|---|
| o.b.® ProComfort tampon Normal Absorbency, 9-12 g, N = 10 | $0.216 \pm 0.008$ | $0.435 \pm 0.024$ | $0.378 \pm 0.023$ |
| "High Fiber density" Tampon Prototype Normal Absorbency, 9-12 g, N = 10 | $0.275 \pm 0.014$ | $0.507 \pm 0.036$ | $0.410 \pm 0.021$ |
| t-test ProComfort tampon vs Prototype | $p = 0.005$ | $p < 0.0001$ | $p < 0.0001$ |

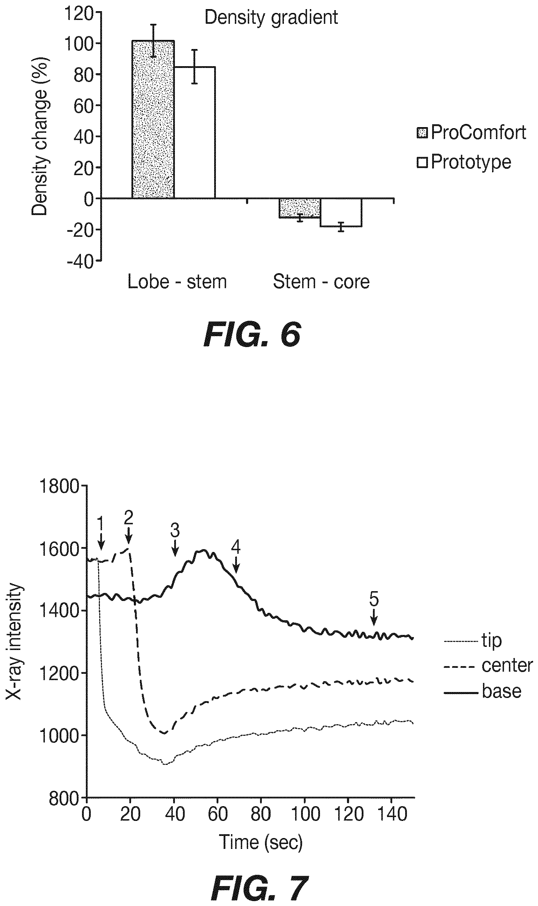

While the absolute fiber density values are informative, it is the fiber density gradient that impacts the flow of fluid from one region of the tampon to another. The gradients between the lobe and the stem, and the stem and the core, are shown in Figure 6. These fiber density gradients are significantly different between the two tampon types, for both the lobe-stem ($p = 0.002$) and the stem-core ($p < 0.0001$) transitions.

CT scans of tampons demonstrated that the density of material inside the tampons could be measured accurately, using a non-invasive and non-destructive imaging methodology. Tampons exhibited X-ray densities ranging from -750 HU to -430 HU. Conversion factors were derived from standard pellets of known fiber density, to relate X-ray densities in HU to mass densities in g/cc.

Two different tampon types were tested, to compare and contrast the performance of a commercially available o.b.® ProComfort tampon, and a High Fiber density Prototype tampon. Based on CT imaging data, three main differences can be observed between the o.b.® ProComfort tampon and the Prototype tampon: (1) the o.b.® ProComfort tampon showed lower fiber density at the lobe, stem, and core; (2) o.b.® ProComfort tampon showed a higher fiber density gradient between the lobe and stem versus the Prototype tampon; (3) both tampon types exhibited a negative gradient between the stem and the core, although the o.b.® ProComfort tampon had a smaller gradient than the Prototype.

A consumer use study on the o.b.® ProComfort tampon versus the Prototype tampon with higher fiber density was conducted with results showing significantly higher leakage rates for the Prototype tampon, see Table 2, below.

**Table 2: Results of a tampon leakage consumer use study showing the percentage of tampons that were reported to leak during the test.**

| | Cell 1: Current o.b. ® ProComfort tampon Normal Absorbency (9-12 g) | Cell 2: High Fiber Density Tampon Prototype Normal Absorbency (9-12 g) |
|---|---|---|
| Base size (tampons) | 664 | 655 |
| % of tampon leakage | **20.8†** | **24.7†** |
| † *Significant difference at 90% Confidence Level* | | |

The consumer use test showed that the high fiber density tampon prototype performed poorly. The higher leakage rate may be attributed to the fiber density gradient of the Prototype tampon. The fiber density gradient from the lobe to the stem was lower in the Prototype tampon than the o.b.® ProComfort tampon, which may slow fluid transfer away from the periphery. In addition, there was a negative fiber density gradient from the stem to the core, particularly in the Prototype tampon, which could prevent fluid moving into the core from the stem. In both areas, the fluid flow is sub-optimal, and the o.b.® ProComfort tampon outperformed the Prototype.

Dynamic Fluoroscope:

Tampons were imaged using dynamic fluoroscope (Siemens MicroCAT II). Acquisition parameters included a 70 kVp X-ray voltage at 500 uA current, a 100 ms exposure time, with 50 um pixels.

The tampon overwrap was carefully removed from each tampon before testing. The tampon weight was recorded in order to calculate the total fluid add-on. Each tampon was placed to the left side of the simulated vaginal model made out of a thermoplastic elastomeric polymer (Dermasol, California Medical Innovations) in an environment that instilled uniform intra-abdominal pressure shown in Fig. 2. The intra-abdominal pressure was instilled using an infusion pump at a constant pressure of 25 cm H20 $\pm$ 5 cm H20. K-Y® jelly (Reckitt Benckiser (North America) Inc., Parsippany, New Jersey, USA) was used on the tip of the tampon for ease of insertion (approximately 0.28g).

5 cc of a simulated menstrual fluid was instilled into the pressurized chamber (test container 106) through port 206c over 20 seconds (similar to a gush) via a syringe by hand. Dynamic fluoroscopic data was recorded over 5 minutes as the tampon absorbed the fluid. Scans were taken during the menstrual fluid insult of the tampon at a rate of one image per 1.5 second.

Dynamic Fluoroscopy Test Samples (In-vitro):

Five tampons of each o.b.® Original (McNeil-PPC, Inc., Skillman, New Jersey, US) in Super Absorbency and o.b.® Flexia (J&J GmbH, Wuppertal, Germany) in Super Absorbency were imaged during the menstrual fluid insult, as described above. Fluid movement through the tampon was visualized in the sagittal view.

Clinical Test Samples (In-vivo):

Five (5) women used the o.b.® Original tampon (J&J GmbH, Wuppertal, Germany) in the Normal absorbency versus the o.b.® Flexia tampon (J&J GmbH, Wuppertal, Germany) in Normal absorbency in a home use test. Each subject recorded the stain pattern of the tampon post-wear in a tampon diary sheet.

**Results**

The stain pattern data from the in-vivo clinical test samples were compared against the stain patterns achieved during the in-vitro dynamic fluoroscope insults. Both in-vivo and in-vitro data sets showed similar stain patterns for the o.b.® Original tampons - the absorbed fluid (both menstrual in in-vivo testing and test fluid in in-vitro testing) remains to one side of the tampon in the sagittal view. Similarly, in-vivo and in-vitro data sets showed similar stain patterns for the o.b.® Flexia tampons - the absorbed fluid (both menstrual in in-vivo testing and test fluid in in-vitro testing) is distributed around the tampon. This movement is facilitated by the apertured film that is attached to the tampon in a wing-like design.
Fig. 7 shows the x-ray intensity data, in a 2-dimensional view through the tampon, as test fluid flowed from the tip of the tampon to its base over time. As fluid was introduced into the tip, the X-ray intensity rapidly decreased due to absorption of the X-ray beam by the fluid (#1). Approximately 12 sec after the fluid passed through the tip, it reached the center, and caused the X-ray intensity to drop (#2). Another 10 sec after that, the X-ray intensity in the base appeared to increase, indicating that, before the fluid arrived, the material in the base of the tampon was expanding and the density decreasing (#3). At 60-80 sec, the fluid reached the base, and the X-ray intensity dropped (#4). Fluid density throughout the tampon reached equilibrium after about 2 min, and the X-ray intensity remained constant (#5).
The specification and embodiments above are presented to aid in the complete and nonlimiting understanding of the invention disclosed herein. Since many variations and embodiments of the invention can be made without departing from its scope, the invention resides in the claims hereinafter appended.

**Claims**

1. Apparatus for in vitro testing of a tampon comprising:

    a) a radiolucent pressure chamber comprising:

        i) a radiolucent simulated body cavity arranged and configured to accommodate the tampon, wherein the radiolucent simulated body cavity comprises a vaginal model;
        ii) a test liquid port; and
        iii) a pressure control system;

    b) a radiological device for collecting radiological data from the tampon having:

        i) a radiolucent test device support capable of accommodating the radiolucent pressure chamber; and
        ii) at least one radio scanning element rotatable about the radiolucent test device support; and

    c) a computing device operatively connected to the radiological device having a program for analyzing the data collected by the radiological device.

2. A method for in vitro testing of a tampon comprising the steps of:

a) inserting the tampon into a radiolucent simulated body cavity, wherein the radiolucent simulated body cavity comprises a vaginal model;

b) placing the radiolucent simulated body cavity into a radiolucent pressure chamber comprising;

    i) a simulated body cavity arranged and configured to accommodate the tampon;
    ii) a test liquid port; and
    iii) a pressure control system;

c) placing the radiolucent pressure chamber onto a radiolucent test device support in a radiological device for collecting radiological data from the tampon having at least one radio scanning element rotatable about the radiolucent test device support;

d) pressurizing the radiolucent pressure chamber to a predetermined pressure;

e) providing test liquid through the test liquid port and to the tampon;

f) acquiring data relating to the tampon and the test liquid via the radiological device and an associated computing device; and

g) analyzing the acquired data.

**3.** The method according to Claim 2, wherein predetermined pressure of the radiolucent pressure chamber is between about 20 to about 50 cm H2O.

**Patentansprüche**

**1.** In-vitro-Testgerät für einen Tampon, umfassend:

    a) eine strahlendurchlässige Druckkammer, umfassend:

        i) eine strahlendurchlässige simulierte Körperhöhle, die zur Aufnahme des Tampons angeordnet und ausgelegt ist, wobei die strahlendurchlässige simulierte Körperhöhle ein Vaginamodell umfasst;
        ii) eine Testflüssigkeitsöffnung; und
        iii) ein Druckkontrollsystem;

    b) eine radiologische Vorrichtung zum Sammeln von radiologischen Daten aus dem Tampon, mit:

        i) einem strahlendurchlässigen Testgeräteträger, der die strahlendurchlässige Druckkammer aufnehmen kann; und
        ii) mindestens ein Radio-Scanning-Element, das um den strahlendurchlässigen Testgeräteträger drehbar ist; und

    c) eine Computervorrichtung, die mit der radiologischen Vorrichtung in Betriebsverbindung steht und ein Programm zur Analyse der von der radiologischen Vorrichtung gesammelten Daten aufweist.

**2.** In-vitro-Testverfahren für einen Tampon, umfassend die folgenden Schritte:

    a) Einführen des Tampons in eine strahlendurchlässige simulierte Körperhöhle, wobei die die strahlendurchlässige simulierte Körperhöhle ein Vaginamodell umfasst;

    b) Platzieren der strahlendurchlässigen simulierten Körperhöhle in einer strahlendurchlässigen Druckkammer, umfassend:

        i) eine simulierte Körperhöhle, die zur Aufnahme des Tampons angeordnet und ausgelegt ist;
        ii) eine Testflüssigkeitsöffnung; und
        iii) ein Druckkontrollsystem;

    c) Platzieren der strahlendurchlässigen Druckkammer auf einem strahlendurchlässigen Testgeräteträger in einer radiologischen Vorrichtung zum Sammeln von radiologischen Daten aus dem Tampon, die mindestens ein Radio-Scanning-Element aufweist, das um den strahlendurchlässigen Testgeräteträger drehbar ist;

    d) Druckbeaufschlagen der strahlendurchlässigen Druckkammer auf einen vorbestimmten Druck;

    e) Bereitstellen einer Testflüssigkeit durch die Testflüssigkeitsöffnung und zum Tampon;

f) Erfassen von Daten in Bezug auf den Tampon und die Testflüssigkeit über die radiologische Vorrichtung und eine dazugehörige Computervorrichtung; und

g) Auswerten der erfassten Daten.

**3.** Verfahren nach Anspruch 2, wobei der vorbestimmte Druck der strahlendurchlässigen Druckkammer zwischen ungefähr 20 und ungefähr 50 cm H2O beträgt.

**Revendications**

**1.** Appareil de test in vitro d'un tampon comprenant :

a) une chambre de pression radio-transparente comprenant :

i) une cavité corporelle simulée radio-transparente agencée et configurée pour recevoir le tampon, la cavité corporelle simulée radio-transparente comprenant un modèle vaginal ;
ii) un orifice de liquide de test ; et
iii) un système de régulation de pression ;

b) un dispositif radiologique pour collecter des données radiologiques à partir du tampon ayant :

i) un support de dispositif de test radio-transparent pouvant recevoir la chambre de pression radio-transparente ; et
ii) au moins un élément de balayage radio pouvant tourner autour du support de dispositif de test radio-transparent ; et

c) un dispositif informatique connecté fonctionnellement au dispositif radiologique, ayant un programme pour analyser les données collectées par le dispositif radiologique.

**2.** Procédé de test in vitro d'un tampon comprenant les étapes consistant à :

a) insérer le tampon dans une cavité corporelle simulée radio-transparente, la cavité corporelle simulée radio-transparente comprenant un modèle vaginal ;
b) placer la cavité corporelle simulée radio-transparente dans une chambre de pression radio-transparente comprenant ;

i) une cavité corporelle simulée, agencée et configurée pour recevoir le tampon ;
ii) un orifice de liquide de test ; et
iii) un système de régulation de pression ;

c) placer la chambre de pression radio-transparente sur un support de dispositif de test radio-transparent dans un dispositif radiologique pour collecter des données radiologiques provenant du tampon, ayant au moins un élément de balayage radio pouvant tourner autour du support de dispositif de test radio-transparent ;
d) mettre sous pression la chambre de pression radio-transparente à une pression prédéterminée ;
e) fournir un liquide de test à travers l'orifice de liquide de test et au tampon ;
f) acquérir des données relatives au tampon et au liquide de test par l'intermédiaire du dispositif radiologique et d'un dispositif informatique associé ; et
g) analyser les données acquises.

**3.** Procédé selon la revendication 2, la pression prédéterminée de la chambre de pression radio-transparente étant comprise entre environ 20 et environ 50 cm de $H_2O$.

**FIG. 1**

**FIG. 3**

**FIG. 2A**

**FIG. 2B**

**FIG. 2C**

**FIG. 4**

**FIG. 5**

**FIG. 6**

**FIG. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6839402 B
- US 7166085 B
- US 20120277710 A
- US 20110200976 A1